# EUROPEAN PATENT SPECIFICATION

(11) **EP 4 346 754 B1**
(45) Date of publication and mention of the grant of the patent: **19.08.2026**
(21) Application number: 22727633.4
(22) Date of filing: 12.05.2022
(51) Int. Cl.: A61K 8/58, A61K 8/73, A61K 8/891, A61K 8/893, A61K 8/894, A61K 8/898, A61Q 5/02, A61Q 5/12, C08B 37/02, C08L 5/02

(54) **SHAMPOO FORMULATION WITH ENHANCED SILICONE DEPOSITION**
SHAMPOO-FORMULIERUNG MIT VERBESSERTER SILIKONABLAGERUNG
SHAMPOOING AVEC UN DÉPÔT DE SILICONE AMÉLIORÉ

(30) Priority: 25.05.2021 US 202163192786 P
(43) Date of publication of application: 10.04.2024
(73) Proprietor: Rohm and Haas Company, Collegeville, PA 19426 (US); Union Carbide Corporation, Seadrift, TX 77983 (US)
(72) Inventor: LEAL, Lyndsay M., Collegeville, Pennsylvania 19426 (US); BAI, Lu, Auburn, Michigan 48611 (US); PARTAIN III, Emmett M., Bound Brook, New Jersey 08805 (US); MILLER, Daniel S., Collegeville, Pennsylvania 19426 (US); GU, Junsi, Collegeville, Pennsylvania 19426 (US); JING, Meng, Collegeville, Pennsylvania 19426 (US)
(74) Representative: Beck Greener LLP
(86) International application number: PCT/US2022/028924
(87) International publication number: WO 2022/250967

(56) References cited:
- WO-A1-2020/101912
- WO-A2-2004/108099
- WO-A2-2010/111576
- US-A1- 2008 003 192
- US-B2- 7 067 499
- SUSAN L JORDAN ET AL: "Effect of Hydrophobic Substitution on Cationic Conditioning Polymers", CHEMISTRY, PROCESS DESIGN, AND SAFETY FOR THE NITRATION INDUSTRY /ACS /SYMPOSIUM SERIES, AMERICAN CHEMICAL SOCIETY/OXFORD UNIVERSITY PRESS, US, vol. 961, 12 April 2007 (2007-04-12), pages 59 - 71, XP009137227, ISSN: 0097-6156, DOI: 10.1021/BK-2007-0961.CH002
- T V DROVETSKAYA ET AL: "New high-charge density hydrophobically modified cationic HEC polymers for improved co-deposition of benefit agents and serious conditioning for problem hair", JOURNAL OF COSMETIC SCIENCE, 31 August 2007 (2007-08-31), pages 421 - 434, XP055235466, Retrieved from the Internet <URL:http://journal.scconline.org//pdf/cc2007/cc058n04/p00421-p00434.pdf> [retrieved on 20151210]

## Description

The present invention relates to a shampoo formulation. In particular, the present invention relates to a shampoo formulation containing: a dermatologically acceptable vehicle; a dermatologically acceptable silicone; a dermatologically acceptable hair cleaning surfactant; a deposition aid polymer, wherein the deposition aid polymer is a modified carbohydrate polymer, comprising a dextran base polymer functionalized with (i) quaternary ammonium groups of formula (II) bound to a pendent oxygen on the dextran base polymer 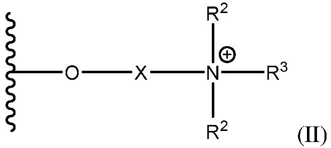 wherein 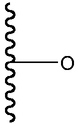 is a pendent oxygen on the dextran base polymer; wherein X is a divalent linking group; wherein each R² is independently selected from the group consisting of a C₁₋₄ alkyl group; and wherein R³ is a linear or branched C₁₋₁₆ alkyl group; and (ii) hydrophobic substituents of formula (III) bound to a pendent oxygen on the dextran base polymer 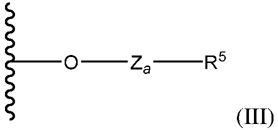 wherein 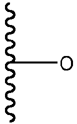 is a pendent oxygen on the dextran base polymer; wherein Z is a divalent linking group; wherein *a* is 0 or 1; and wherein each R⁵ is a linear or branched C₆₋₂₂ alkyl group.

Hair cleansing has become a ubiquitous component of personal hygiene. Cleansing of the hair facilitates the removal of dirt, germs and other things that are perceived as harmful to the hair or the individual. Cleansing formulations typically include a surfactant to promote the removal of materials deposited on the hair. Unfortunately, the cleansing formulations remove both undesirable and desirable materials from hair. For example, cleansing formulations frequently undesirably remove oils from hair; oils operate to protect hair from loss of moisture. Removal of too much oil from hair may leave the hair vulnerable to becoming dry and damaged. One solution to this concern is the selection of mild surfactants. Another approach is to incorporate additives that help replace the oils removed through deposition; however, this approach has proven difficult in implementation, particularly in rinse off applications.

In U.S. Patent No. 7,067,499, Erazo-Majewicz, et al. disclose personal care and household care product composition comprising at least one cationic polygalactomannan or a derivative of cationic polygalactomannans wherein the derivative moiety on the cationic derivatized polygalactomannan is selected from the group consisting of alkyl, hydroxyalkyl, alkylhydroxyalkyl, and carboxymethyl wherein the alkyl has a carbon chain containing from 1 to 22 carbons and the hydroxyalkyl is selected from the group consisting of hydroxyethyl, hydroxypropyl, and hydroxybutyl, wherein the at least one cationic polygalactomannan or derivative of cationic polygalactomannans have a mean average molecular weight (Mw) having a lower limit of 5,000 and an upper limit of 200,000 and having a light transmittance in a 10% aqueous solution of greater than 80% at a light wavelength of 600 nm and a protein content of less than 1.0% by weight of polysaccharide, and aldehyde functionality content of at least 0.01 meq/gram.

While conventionally used deposition aids such as soluble cationic modified celluloses (e.g., polyquaternium-10), guar hydroxypropyltrimonium chloride and other cationic polymers (e.g., polyquaternium-6, polyquaternium-7) provide a certain level of deposition in personal care cleansers; they nevertheless exhibit low efficiency necessitating a relatively high incorporation of the active into the personal care cleanser formulation to facilitate desired results. Such high active (e.g., silicone) levels, however, detrimentally effect the foam/lathery in use consumer feel of the formulation and cost.

Accordingly, there remains a need for deposition aids that facilitate enhanced efficiency of silicone deposition from shampoo formulations. There is also a continuing need for new deposition aids having an increased natural origin index (ISO16128) when compared with conventional deposition aids.

The present invention provides a shampoo formulation, comprising: a dermatologically acceptable vehicle; a dermatologically acceptable silicone; a dermatologically acceptable hair cleaning surfactant; a deposition aid polymer, wherein the deposition aid polymer is a modified carbohydrate polymer, comprising a dextran base polymer functionalized with (i) quaternary ammonium groups of formula (II) bound to a pendent oxygen on the dextran base polymer 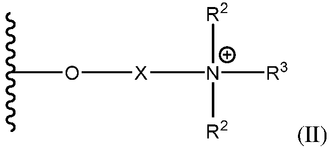 wherein 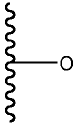 is a pendent oxygen on the dextran base polymer; wherein X is a divalent linking group; wherein each R² is independently selected from the group consisting of a C₁₋₄ alkyl group; and wherein R³ is a linear or branched C₁₋₁₆ alkyl group; and (ii) hydrophobic substituents of formula (III) bound to a pendent oxygen on the dextran base polymer 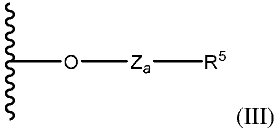 wherein 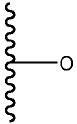 is a pendent oxygen on the dextran base polymer; wherein Z is a divalent linking group; wherein *a* is 0 or 1; and wherein each R⁵ is a linear or branched C₆₋₂₂ alkyl group.

The present invention provides a method of depositing silicone on to mammalian hair, comprising: selecting a shampoo formulation of the present invention; and applying the shampoo formulation to mammalian hair; wherein the deposition aid polymer enhances the deposition of the dermatologically acceptable silicone from the shampoo formulation onto the mammalian hair relative to an otherwise identical formulation without the deposition aid polymer.

### DETAILED DESCRIPTION

We have surprisingly found that silicone deposition from shampoo formulations can be enhanced through incorporation of a deposition aid polymer, wherein the deposition aid polymer is a modified carbohydrate polymer, comprising a dextran base polymer functionalized with (i) quaternary ammonium groups of formula (II) bound to a pendent oxygen on the dextran base polymer 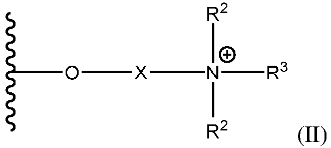 wherein 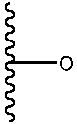 is a pendent oxygen on the dextran base polymer; wherein X is a divalent linking group; wherein each R² is independently selected from the group consisting of a C₁₋₄ alkyl group; and wherein R³ is a linear or branched C₁₋₁₆ alkyl group; and (ii) hydrophobic substituents of formula (III) bound to a pendent oxygen on the dextran base polymer 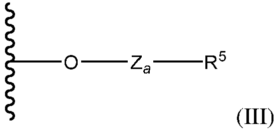 wherein 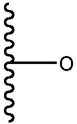 is a pendent oxygen on the dextran base polymer; wherein Z is a divalent linking group; wherein *a* is 0 or 1; and wherein each R⁵ is a linear or branched C₆₋₂₂ alkyl group.

Unless otherwise indicated, ratios, percentages, parts, and the like are by weight.

As used herein, unless otherwise indicated, the phrase "molecular weight" or M_{W} refers to the weight average molecular weight as measured in a conventional manner with gel permeation chromatography (GPC) and conventional standards, such as polyethylene glycol standards. GPC techniques are discussed in detail in Modern Size Exclusion Chromatography, W. W. Yau, J. J. Kirkland, D. D. Bly; Wiley-Interscience, 1979, and in A Guide to Materials Characterization and Chemical Analysis, J. P. Sibilia; VCH, 1988, p.81-84. Molecular weights are reported herein in units of Daltons, or equivalently, g/mol.

The term "dermatologically acceptable" as used herein and in the appended claims refers to ingredients that are typically used for topical application to the skin or hair, and is intended to underscore that materials that are toxic when present in the amounts typically found in hair care compositions are not contemplated as part of the present invention.

Preferably, the shampoo formulation of the present invention is selected from the group consisting of a shampoo and a conditioning shampoo.

Preferably, the shampoo formulation of the present invention, comprises: a dermatologically acceptable vehicle (preferably, 25 to 99.885 wt% (more preferably, 45 to 98.82 wt%; still more preferably, 79 to 97.15 wt%; most preferably, 84 to 94.4 wt%), based on weight of the shampoo formulation, of the dermatologically acceptable vehicle); a dermatologically acceptable silicone (preferably, 0.1 to 5 wt% (more preferably, 0.15 to 4 wt%; still more preferably, 0.25 to 2 wt%; most preferably, 0.4 to 1.5 wt%), based on weight of the shampoo formulation, of the dermatologically acceptable silicone); a dermatologically acceptable hair cleaning surfactant (preferably, 0.01 to 74.899 wt% (more preferably, 1 to 54.84 wt%; still more preferably, 2.5 to 20.65 wt%; most preferably, 5 to 15.4 wt%), based on weight of the shampoo formulation, of the dermatologically acceptable hair cleaning surfactant); and a deposition aid polymer (preferably, 0.005 to 5 wt% (more preferably, 0.03 to 2 wt%; still more preferably, 0.1 to 1 wt%; most preferably, 0.2 to 0.4 wt%), based on weight of the shampoo formulation, of the deposition aid polymer), wherein the deposition aid polymer is a modified carbohydrate polymer, comprising a dextran base polymer functionalized with (i) quaternary ammonium groups of formula (II) bound to a pendent oxygen on the dextran base polymer 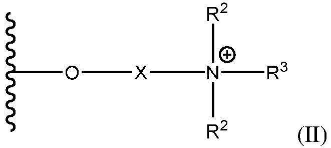 wherein 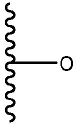 is a pendent oxygen on the dextran base polymer; wherein X is a divalent linking group; wherein each R² is independently selected from the group consisting of a C₁₋₄ alkyl group; and wherein R³ is a linear or branched C₁₋₁₆ alkyl group; and (ii) hydrophobic substituents of formula (III) bound to a pendent oxygen on the dextran base polymer 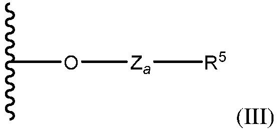 wherein 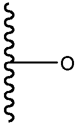 is a pendent oxygen on the dextran base polymer; wherein Z is a divalent linking group; wherein *a* is 0 or 1; and wherein each R⁵ is a linear or branched C₆₋₂₂ alkyl group (preferably, wherein the dextran base polymer has a weight average molecular weight of 1,000 to 3,000,000 Daltons) (preferably; wherein the deposition aid polymer enhances deposition of silicone from the shampoo formulation onto mammalian hair (preferably, human hair)).

Preferably, the shampoo formulation of the present invention is a liquid formulation. More preferably, the shampoo formulation of the present invention is an aqueous liquid formulation.

Preferably, the shampoo formulation of the present invention, comprises: 25 to 99.885 wt% (more preferably, 45 to 98.82 wt%; still more preferably, 79 to 97.15 wt%; most preferably, 84 to 94.4 wt%), based on weight of the shampoo formulation, of a dermatologically acceptable vehicle. Still more preferably, the shampoo formulation of the present invention, comprises: 25 to 99.885 wt% (more preferably, 45 to 98.82 wt%; still more preferably, 79 to 97.15 wt%; most preferably, 84 to 94.4 wt%), based on weight of the shampoo formulation, of a dermatologically acceptable vehicle; wherein the dermatologically acceptable vehicle comprises water. Yet more preferably, the shampoo formulation of the present invention, comprises: 25 to 99.885 wt% (more preferably, 45 to 98.82 wt%; still more preferably, 79 to 97.15 wt%; most preferably, 84 to 94.4 wt%), based on weight of the shampoo formulation, of a dermatologically acceptable vehicle; wherein the dermatologically acceptable vehicle is selected from the group consisting of water and an aqueous C₁₋₄ alcohol mixture. Most preferably, the shampoo formulation of the present invention, comprises: 25 to 99.885 wt% (more preferably, 45 to 98.82 wt%; still more preferably, 79 to 97.15 wt%; most preferably, 84 to 94.4 wt%), based on weight of the shampoo formulation, of a dermatologically acceptable vehicle; wherein the dermatologically acceptable vehicle is water.

Preferably, the water used in the shampoo formulation of the present invention is at least one of distilled water and deionized water. More preferably, the water used in the shampoo formulation of the present invention is distilled and deionized.

Preferably, the shampoo formulation of the present invention comprises 0.1 to 5 wt% (preferably, 0.15 to 4 wt%; more preferably, 0.25 to 2 wt%; most preferably, 0.4 to 1.5 wt%), based on weight of the shampoo formulation, of a dermatologically acceptable silicone (preferably, wherein the dermatologically acceptable silicone conditions hair). More preferably, the shampoo formulation of the present invention comprises 0.1 to 5 wt% (preferably, 0.15 to 4 wt%; more preferably, 0.25 to 2 wt%; most preferably, 0.4 to 1.5 wt%), based on weight of the shampoo formulation, of a dermatologically acceptable silicone, wherein the dermatologically acceptable silicone is selected from the group consisting of amodimethicone, cyclomethicone, dimethicone, dimethiconol, hexadecyl methicone, hexamethyldisiloxane, bis-diisopropanol amino-PG-propyl disiloxane, methicone, phenyl dimethicone, bis-vinyl dimethicone, stearoxy dimethicone polyalkyl siloxane, polyalkylaryl siloxane, silicone gums (i.e., polydiorganosiloxanes having a weight average molecular weight of 200,000 to 1,000,000 Daltons), polyaminofunctional silicones (e.g., Dow Corning^{®} 929) and mixtures thereof. Yet more preferably, the shampoo formulation of the present invention comprises 0.1 to 5 wt% (preferably, 0.15 to 4 wt%; more preferably, 0.25 to 2 wt%; most preferably, 0.4 to 1.5 wt%), based on weight of the shampoo formulation, of a dermatologically acceptable silicone, wherein the dermatologically acceptable silicone is selected from the group consisting of amodimethicone, cyclomethicone, dimethicone, dimethiconol, hexadecyl methicone, hexamethyldisiloxane, methicone, phenyl dimethicone, stearoxy dimethicone and mixtures thereof. Still yet more preferably, the shampoo formulation of the present invention comprises 0.1 to 5 wt% (preferably, 0.15 to 4 wt%; more preferably, 0.25 to 2 wt%; most preferably, 0.4 to 1.5 wt%), based on weight of the shampoo formulation, of a dermatologically acceptable silicone, wherein the dermatologically acceptable silicone is selected from the group consisting of amodimethicone, cyclomethicone, dimethicone, dimethiconol, hexadecyl methicone, methicone and mixtures thereof. Still more preferably, the shampoo formulation of the present invention comprises 0.1 to 5 wt% (preferably, 0.15 to 4 wt%; more preferably, 0.25 to 2 wt%; most preferably, 0.4 to 1.5 wt%), based on weight of the shampoo formulation, of a dermatologically acceptable silicone, wherein the dermatologically acceptable silicone is selected from the group consisting of amodimethicone, dimethicone, dimethiconol and a mixture thereof. Most preferably, the shampoo formulation of the present invention comprises 0.1 to 5 wt% (preferably, 0.15 to 4 wt%; more preferably, 0.25 to 2 wt%; most preferably, 0.4 to 1.5 wt%), based on weight of the shampoo formulation, of a dermatologically acceptable silicone, wherein the dermatologically acceptable silicone comprises a dimethiconol.

Preferably, the shampoo formulation of the present invention comprises 0.01 to 74.899 wt% (preferably, 1 to 54.84 wt%; more preferably, 2.5 to 20.65 wt%; most preferably, 5 to 15.4 wt%), based on weight of the shampoo formulation, of a dermatologically acceptable hair cleaning surfactant. More preferably, the shampoo formulation of the present invention comprises 0.01 to 74.899 wt% (preferably, 1 to 54.84 wt%; more preferably, 2.5 to 20.65 wt%; most preferably, 5 to 15.4 wt%), based on weight of the shampoo formulation, of a dermatologically acceptable hair cleaning surfactant; wherein the dermatologically acceptable hair cleaning surfactant is selected from the group consisting of alkyl polyglucosides (e.g., lauryl glucoside, coco-glucoside, decyl glucoside), glycinates (e.g., sodium cocoyl glycinate), betaines (e.g., alkyl betaines such as cetyl betaine and amido betaines such as cocamidopropyl betaine), taurates (e.g., sodium methyl cocoyl taurate), glutamates (e.g., sodium cocoyl glutamate), sarcosinates (e.g., sodium lauroyl sarcosinate), isethionates (e.g., sodium cocoyl isethionate, sodium lauroyl methyl isethionate), sulfoacetates (e.g., sodium lauryl sulfoacetate), alaninates (e.g., sodium cocoyl alaninate), amphoacetates (e.g., sodium cocoamphoacetate), sulfates (e.g., sodium lauryl ether sulfate (SLES)), sulfonates (e.g., sodium C₁₄₋₁₆ olefin sulfonate), succinates (e.g., disodium lauryl sulfosuccinate), fatty alkanolamides (e.g., cocamide monoethanolamine, cocamide diethanolamine, soyamide diethanolamine, lauramide diethanolamine, oleamide monoisopropanolamine, stearamide monoethanolamine, myristamide monoethanolamine, lauramide monoethanolamine, capramide diethanolamine, ricinoleamide diethanolamine, myristamide diethanolamine, stearamide diethanolamine, oleylamide diethanolamine, tallowamide diethanolamine, lauramide monoisopropanolamine, tallowamide monoethanolamine, isostearamide diethanolamine, isostearamide monoethanolamine) and mixtures thereof. Still more preferably, the shampoo formulation of the present invention comprises 0.01 to 74.899 wt% (preferably, 1 to 54.84 wt%; more preferably, 2.5 to 20.65 wt%; most preferably, 5 to 15.4 wt%), based on weight of the shampoo formulation, of a dermatologically acceptable hair cleaning surfactant; wherein the dermatologically acceptable hair cleaning surfactant includes a sodium lauryl ether sulfate. Yet more preferably, the shampoo formulation of the present invention comprises 0.01 to 74.899 wt% (preferably, 1 to 54.84 wt%; more preferably, 2.5 to 20.65 wt%; most preferably, 5 to 15.4 wt%), based on weight of the shampoo formulation, of a dermatologically acceptable hair cleaning surfactant; wherein the dermatologically acceptable hair cleaning surfactant includes a sodium lauryl ether sulfate; wherein the sodium lauryl ether sulfate is selected from the group consisting of a sodium lauryl ether sulfate having an average of one -(OCH₂CH₂)- group per molecule, a sodium lauryl ether sulfate having an average of two -(OCH₂CH₂)- groups per molecule, a sodium lauryl ether sulfate having an average of three -(OCH₂CH₂)- groups per molecule and mixtures thereof (preferably, wherein the sodium lauryl ether sulfate is selected from the group consisting of a sodium lauryl ether sulfate having an average of two -(OCH₂CH₂)-groups per molecule, a sodium lauryl ether sulfate having an average of three -(OCH₂CH₂)-groups per molecule and mixtures thereof; more preferably, wherein the sodium lauryl ether sulfate is a sodium lauryl ether sulfate having an average of two -(OCH₂CH₂)- groups per molecule). Most preferably, the shampoo formulation of the present invention comprises 0.01 to 74.899 wt% (more preferably, 1 to 54.84 wt%; still more preferably, 2.5 to 20.65 wt%; most preferably, 5 to 15.4 wt%), based on weight of the shampoo formulation, of a dermatologically acceptable hair cleaning surfactant; wherein the dermatologically acceptable hair cleaning surfactant includes a blend of a sodium lauryl ether sulfate, a cocamide monoethanolamine and a cocamidopropyl betaine (preferably, wherein the sodium lauryl ether sulfate is selected from the group consisting of a sodium lauryl ether sulfate having an average of one -(OCH₂CH₂)- group per molecule, a sodium lauryl ether sulfate having an average of two -(OCH₂CH₂)- groups per molecule, a sodium lauryl ether sulfate having an average of three -(OCH₂CH₂)- groups per molecule and mixtures thereof; more preferably, wherein the sodium lauryl ether sulfate is selected from the group consisting of a sodium lauryl ether sulfate having an average of two -(OCH₂CH₂)- groups per molecule, a sodium lauryl ether sulfate having an average of three -(OCH₂CH₂)- groups per molecule and mixtures thereof; most preferably, wherein the sodium lauryl ether sulfate has an average of two -(OCH₂CH₂)- groups per molecule).

Preferably, the shampoo formulation of the present invention comprises 0.005 to 5 wt% (more preferably, 0.03 to 2 wt%; still more preferably, 0.1 to 1 wt%; most preferably, 0.2 to 0.4 wt%), based on weight of the shampoo formulation, of a deposition aid polymer; wherein the deposition aid polymer is a modified carbohydrate polymer, comprising a dextran base polymer functionalized with (i) quaternary ammonium groups of formula (II) bound to a pendent oxygen on the dextran base polymer; and (ii) hydrophobic substituents of formula (III) bound to a pendent oxygen on the dextran base polymer; wherein the dextran base polymer has a weight average molecular weight of 1,000 to 3,000,000 Daltons.

Preferably, the dextran base polymer has a weight average molecular weight of 1,000 to 3,000,000 Daltons (preferably, 50,000 to 2,000,000 Daltons; more preferably, 100,000 to 1,000,000 Daltons; still more preferably, 125,000 to 800,000 Daltons; most preferably, 145,000 to 525,000 Daltons). More preferably, the dextran base polymer has a weight average molecular weight of 1,000 to 3,000,000 Daltons (preferably, 50,000 to 2,000,000 Daltons; more preferably, 100,000 to 1,000,000 Daltons; still more preferably, 125,000 to 800,000 Daltons; most preferably, 145,000 to 525,000 Daltons); and the dextran base polymer is a branched chain dextran polymer comprising a plurality of glucose structural units; wherein 90 to 98 mol% (preferably, 92.5 to 97.5 mol%; more preferably, 93 to 97 mol%; most preferably, 94 to 96 mol%) of the glucose structural units are connected by α-D-1,6 linkages and 2 to 10 mol% (preferably, 2.5 to 7.5 mol%; more preferably, 3 to 7 mol%; most preferably, 4 to 6 mol%) of the glucose structural units are connected by α-1,3 linkages. Most preferably, the dextran base polymer has a weight average molecular weight of 1,000 to 3,000,000 Daltons (preferably, 50,000 to 2,000,000 Daltons; more preferably, 100,000 to 1,000,000 Daltons; still more preferably, 125,000 to 800,000 Daltons; most preferably, 145,000 to 525,000 Daltons); and the dextran base polymer is a branched chain dextran polymer comprising a plurality of glucose structural units; wherein 90 to 98 mol% (preferably, 92.5 to 97.5 mol%; more preferably, 93 to 97 mol%; most preferably, 94 to 96 mol%) of the glucose structural units are connected by α-D-1,6 linkages and 2 to 10 mol% (preferably, 2.5 to 7.5 mol%; more preferably, 3 to 7 mol%; most preferably, 4 to 6 mol%) of the glucose structural units are connected by α-1,3 linkages according to formula I 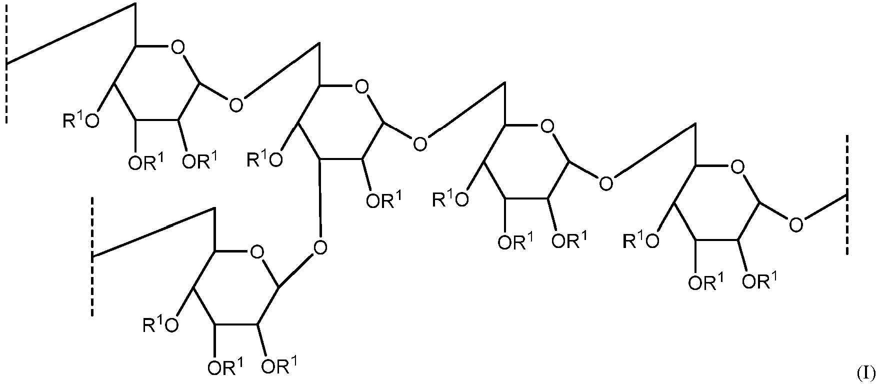 wherein R¹ is selected from a hydrogen, a C₁₋₄ alkyl group and a hydroxy C₁₋₄ alkyl group; and wherein the average branch off the dextran polymer backbone is ≤ 3 anhydroglucose units.

Preferably, the dextran base polymer contains less than 0.01 wt%, based on weight of the dextran base polymer, of alternan. More preferably, the dextran base polymer contains less than 0.001 wt%, based on weight of the dextran base polymer, of alternan. Most preferably, the dextran base polymer contains less than the detectable limit of alternan.

Preferably, the deposition aid polymer is a modified carbohydrate polymer, comprising a dextran base polymer functionalized with (i) quaternary ammonium groups of formula (II) bound to a pendent oxygen on the dextran base polymer 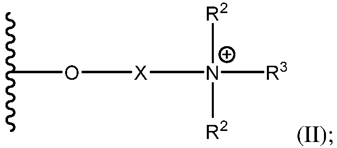 and (ii) hydrophobic substituents of formula (III) bound to a pendent oxygen on the dextran base polymer 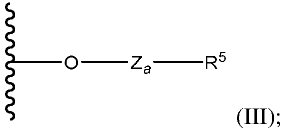 wherein 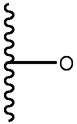 is a pendent oxygen on the dextran base polymer; wherein X is a divalent linking group (preferably, wherein X is selected from divalent alkyl groups, which may optionally be substituted with a hydroxy group, an alkoxy group and/or an ether group; more preferably, wherein X is a -CH₂CH(OR⁴)CH₂- group, where R⁴ is selected from the group consisting of a hydrogen and a linear or branched C₁₋₄ alkyl group; most preferably, wherein X is a -CH₂CH(OH)CH₂- group); wherein each R² is independently selected from a linear or branched C₁₋₄ alkyl group (preferably, a linear or branched C₁₋₃ alkyl group; more preferably, a C₁₋₂ alkyl group; most preferably, a methyl group); wherein each R³ is independently selected from a linear or branched C₁₋₁₆ alkyl group (preferably, a linear or branched C₁₋₁₂ alkyl group; more preferably, a linear or branched C₁₋₈ alkyl group; still more preferably, a methyl group or a linear or branched C₈ alkyl group; most preferably, a methyl group); wherein Z is a divalent linking group (preferably, wherein Z is selected from divalent alkyl groups, which may optionally be substituted with a hydroxy group, an alkoxy group and/or an ether group; more preferably, wherein Z is a -CH₂CH(OR⁶)CH₂O- group, where R⁶ is selected from the group consisting of a hydrogen and a linear or branched C₁₋₄ alkyl group; most preferably, wherein Z is a -CH₂CH(OH)CH₂O- group); wherein *a* is a 0 or 1 (preferably, 0); and wherein each R⁵ is a linear or branched C₆₋₂₂ alkyl group (preferably, a C₁₀₋₂₀ alkyl group; more preferably, a C₁₂₋₁₈ alkyl group; most preferably, a C₁₆ alkyl group). More preferably, the deposition aid polymer is a modified carbohydrate polymer, comprising a dextran base polymer functionalized with (i) quaternary ammonium groups of formula (IIa) bound to a pendent oxygen on the dextran base polymer 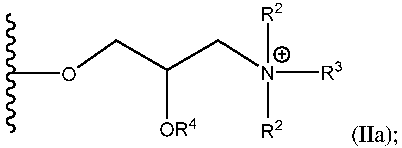 and (ii) hydrophobic substituents of formula (IIIa), formula (IIIb) or both, bound to a pendent oxygen on the dextran base polymer 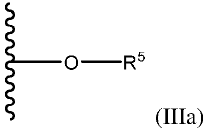 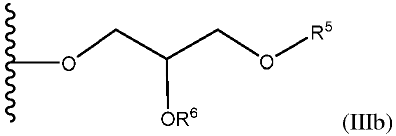 wherein 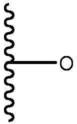 is a pendent oxygen on the dextran base polymer; wherein R⁴ is selected from the group consisting of a hydrogen and a linear or branched C₁₋₄ alkyl group (preferably, R⁴ is a hydrogen); wherein each R² is independently selected from a linear or branched C₁₋₄ alkyl group (preferably, a linear or branched C₁₋₃ alkyl group; more preferably, a C₁₋₂ alkyl group; most preferably, a methyl group); wherein each R³ is independently selected from a linear or branched C₁₋₁₆ alkyl group (preferably, a linear or branched C₁₋₁₂ alkyl group; more preferably, a linear or branched C₁₋₈ alkyl group; still more preferably, a methyl group or a linear or branched C₈ alkyl group; most preferably, a methyl group); wherein each R⁵ is a linear or branched C₆₋₂₂ alkyl group (preferably, a C₁₀₋₂₀ alkyl group; more preferably, a C₁₂₋₁₈ alkyl group; most preferably, a C₁₆ alkyl group); and where R⁶ is selected from the group consisting of a hydrogen and a linear or branched C₁₋₄ alkyl group (preferably, a hydrogen). Most preferably, the deposition aid polymer is a modified carbohydrate polymer, comprising a dextran base polymer functionalized with (i) a quaternary ammonium group of formula (IIa) bound to a pendent oxygen on the dextran base polymer; and (ii) hydrophobic substituents of formula (IIIa) bound to a pendent oxygen on the dextran base polymer; wherein each R² is a methyl group; wherein each R³ is a linear or branched C₁₋₁₂ alkyl group (preferably, a linear or branched C₁₋₈ alkyl group; most preferably, a methyl group or a linear or branched C₈ alkyl group); wherein each R⁴ is a hydrogen; and wherein each R⁵ is a linear or branched C₆₋₂₂ alkyl group (preferably, a C₁₀₋₂₀ alkyl group; more preferably, a C₁₂₋₁₈ alkyl group; most preferably, a C₁₆ alkyl group).

Preferably, the deposition aid polymer has a Kjeldahl nitrogen content, TKN, of 0.5 to 5.0 wt% (preferably, 0.7 to 4 wt%; more preferably, 0.8 to 2.5 wt%; most preferably, 1.4 to 2.0 wt%) measured using a Buchi KjelMaster K-375 automated analyzer, corrected for volatiles and ash measured as described in ASTM method D-2364.

Preferably, the deposition aid polymer has a hydrophobe degree of substitution, DS, of hydrophobic substituents of formula (III) bound to a pendent oxygen on the dextran base polymer of 0.0025 to 0.05 (preferably, 0.003 to 0.04; more preferably, 0.004 to 0.03).

Preferably, the deposition aid polymer comprises < 0.001 meq/gram (preferably, < 0.0001 meq/gram; more preferably, < 0.00001 meq/gram; most preferably, < detectable limit) of aldehyde functionality.

Preferably, the deposition aid polymer comprises < 0.1 % (preferably, < 0.01 %; more preferably, < 0.001 %; most preferably, < detectable limit), of the linkages between individual glucose units in the deposition aid polymer are β-1,4 linkages.

Preferably, the deposition aid polymer comprises < 0.1 % (preferably, < 0.01 %; more preferably, < 0.001 %; most preferably, < detectable limit), of the linkages between individual glucose units in the deposition aid polymer are β-1,3 linkages.

Preferably, the deposition aid polymer comprises < 0.001 meq/gram (preferably, < 0.0001 meq/gram; more preferably, < 0.00001 meq/gram; most preferably, < detectable limit) of silicone containing functionality.

Preferably, the shampoo formulation of the present invention, optionally, further comprises at least one additional ingredient selected from the group consisting of an antimicrobial agent/preservative (e.g., benzoic acid, sorbic acid, phenoxyethanol, methylisothiazolinone); a rheology modifier (e.g., PEG-150 pentaerythrityl tetrastearate); a soap; a colorant; pH adjusting agent; an antioxidant (e.g., butylated hydroxytoluene); a humectant (e.g., glycerin, sorbitol, monoglycerides, lecithins, glycolipids, fatty alcohols, fatty acids, polysaccharides, sorbitan esters, polysorbates (e.g., Polysorbate 20, Polysorbate 40, Polysorbate 60, and Polysorbate 80), diols (e.g., propylene glycol), diol analogs, triols, triol analogs, cationic polymeric polyols); a wax; a foaming agent; an emulsifying agent; a colorant; a fragrance; a chelating agent (e.g., tetrasodium ethylene diamine tetraacetic acid); a preservative (e.g., benzoic acid, sorbic acid, phenoxyethanol, methylisothiazolinone); a bleaching agent; a lubricating agent; a sensory modifier; a sunscreen additive; a vitamin; a protein/amino acid; a plant extract; a natural ingredient; a bioactive agent; an anti-aging agent; a pigment; an acid; a penetrant; an anti-static agent; an anti-frizz agent; an antidandruff agent; a hair waving/straightening agent; a hair styling agent; a hair oil; natural oils or ester emollients (e.g., mono-, di- and tri-glycerides such as sunflower seed oil, coconut oil, cottonseed oil, borage oil, borage seed oil, primrose oil, castor and hydrogenated castor oils, rice bran oil, soybean oil, olive oil, safflower oil, shea butter, jojoba oil and combinations thereof); an absorbent; a hard particle; a soft particle; a conditioning agent (e.g., guar hydroxypropyltrimonium chloride, PQ-10, PQ-7, PQ-67); a slip agent; an opacifier; a pearlizing agent and a salt. More preferably, the shampoo formulation of the present invention, optionally, further comprises at least one additional ingredient selected from the group consisting of an antimicrobial agent/preservative (e.g., benzoic acid, sorbic acid, phenoxyethanol, methylisothiazolinone); a rheology modifier (e.g., PEG-150 pentaerythrityl tetrastearate); and a chelating agent (e.g., tetrasodium ethylene diamine tetraacetic acid). Most preferably, the shampoo formulation of the present invention, optionally, further comprises at least one additional ingredient selected from the group consisting of an antimicrobial agent/preservative mixture of phenoxyethanol and methylisothiazolinone; PEG-150 pentaerythrityl tetrastearate; tetrasodium ethylene diamine tetraacetic acid and a mixture of phenoxyethanol and methylisothiazolinone.

Preferably, the shampoo formulation of the present invention further comprises a thickener. More preferably, the shampoo formulation further comprises a thickener, wherein the thickener is selected to increase the viscosity of the shampoo formulation, preferably without substantially modifying the other properties of the shampoo formulation. Preferably, the shampoo formulation further comprises a thickener, wherein the thickener is selected to increase the viscosity of the shampoo formulation, preferably without substantially modifying the other properties of the shampoo formulation and wherein the thickener accounts for 0 to 5.0 wt% (preferably, 0.1 to 5.0 wt %; more preferably, 0.2 to 2.5 wt %; most preferably, 0.5 to 2.0 wt%), based on weight of the shampoo formulation.

Preferably, the shampoo formulation of the present invention further comprises an antimicrobial agent/preservative. More preferably, the shampoo formulation of the present invention further comprises an antimicrobial/preservative, wherein the antimicrobial/preservative is selected from the group consisting of phenoxyethanol, benzoic acid, benzyl alcohol, sodium benzoate, DMDM hydantoin, 2-ethylhexyl glyceryl ether, isothiazolinone (e.g., methylchloroisothiazolinone, methylisothiazolinone) and mixtures thereof. Still more preferably, the shampoo formulation of the present invention, further comprises an antimicrobial/preservative, wherein the antimicrobial/preservative is a mixture of phenoxyethanol and an isothiazolinone (more preferably, wherein the antimicrobial/preservative is a mixture of phenoxyethanol and methylisothiazolinone).

Preferably, the shampoo formulation of the present invention optionally further comprises a pH adjusting agent. More preferably, the shampoo formulation of the present invention, further comprises a pH adjusting agent, wherein the shampoo formulation has a pH of 4 to 9 (preferably, 4.25 to 8; more preferably, 4.5 to 7; most preferably, 4.75 to 6).

Preferably, the pH adjusting agent is selected from the group consisting of at least one of citric acid, lactic acid, hydrochloric acid, aminoethyl propanediol, triethanolamine, monoethanolamine, sodium hydroxide, potassium hydroxide, amino-2-methyl-1-propanol. More preferably, the pH adjusting agent is selected from the group consisting of at least one of citric acid, lactic acid, sodium hydroxide, potassium hydroxide, triethanolamine, amino-2-methyl-1-propanol. Still more preferably, the pH adjusting agent includes citric acid. Most preferably, the pH adjusting agent is citric acid.

Preferably, the method of depositing silicone onto mammalian hair (preferably human hair) of the present invention, comprises: selecting a shampoo formulation of the present invention and applying the shampoo formulation to mammalian hair; wherein the deposition aid polymer enhances the deposition of the dermatologically acceptable silicone from the shampoo formulation onto the mammalian hair relative to an otherwise identical formulation without the deposition aid polymer. More preferably, the method of depositing silicone on to mammalian hair (preferably human hair) of the present invention, comprises: selecting a shampoo formulation of the present invention, wetting the mammalian hair with water; applying the shampoo formulation to the wetted mammalian hair; and rinsing the shampoo formulation from the mammalian hair with water; wherein the deposition aid polymer enhances the deposition of the dermatologically acceptable silicone from the shampoo formulation onto the mammalian hair relative to an otherwise identical formulation without the deposition aid polymer. Most preferably, the method of depositing silicone on to mammalian hair (preferably human hair) of the present invention, comprises: selecting a shampoo formulation of the present invention, wetting the mammalian hair with water; applying the shampoo formulation to the wetted mammalian hair; and rinsing the shampoo formulation from the mammalian hair with water; wherein dermatologically acceptable silicone present in the shampoo formulation is deposited onto the mammalian hair; and wherein the deposition aid polymer enhances the deposition of the dermatologically acceptable silicone from the shampoo formulation onto the mammalian hair relative to an otherwise identical formulation without the deposition aid polymer.

Some embodiments of the present invention will now be described in detail in the following **Examples.**

### Synthesis S1: Synthesis of Cationic Hexadecyl Modified Dextran Polymer

A 500 mL, four necked, round bottom flask fitted with a rubber serum cap, a nitrogen inlet, a pressure equalizing addition funnel, a stirring paddle and motor, a subsurface thermocouple connected to a J-KEM controller and a Friedrich condenser connected to a mineral oil bubbler was charged with dextran base polymer (20 g; Sigma Aldrich catalog No. D4876) and deionized water (100 g). The contents of the flask were stirred at 70 rpm. While stirring, the head space in the flask was purged with a slow, steady flow of nitrogen (about one bubble per second) for one hour to remove any entrained oxygen in the apparatus. A 50% aqueous sodium hydroxide solution (5.0 g) was then added to the flask contents using a syringe over 2 minutes with continued stirring. After 30 minutes, hexadecyl bromide (15.3 g) was added to the flask contents using a syringe. The addition funnel was charged with 2,3-epoxypropyltrimethylammonium chloride (17.0 g; QUAB^{®} 151 available from SKW QUAB Chemicals), which was then added dropwise to the flask contents over 3 minutes with continued stirring. The flask contents were stirred under nitrogen for an additional 10 minutes, and then heat was applied with a set point temperature of 70 °C using a heating mantle. With continued stirring the flask contents were heated at 70 °C for 3 hours.

The flask contents were then cooled in a water bath while maintaining a positive nitrogen pressure in the flask. A solid polymer product was recovered from the flask contents by non-solvent precipitation in methanol; roughly 1 L of methanol was used for the precipitation of the entire batch. The methanol was decanted off and the polymer recovered was placed in a dish to be dried in vacuo at 50 °C. The dried polymer was manually ground with a mortar and pestle, and screened through a US standard #30 sieve.

The product polymer was obtained as a white solid, with a volatiles content of 4.07%, and ash content (as sodium chloride) of 2.07%, and a Kjeldahl nitrogen content (corrected for ash and volatiles) of 1.507%, corresponding to a cationic substitution, CS, value of 0.217.

### Synthesis S2: Synthesis of Cationic Dodecyl Modified Dextran Polymer

In **Synthesis S2,** cationic dodecyl modified dextran polymer was prepared substantially as described in **Synthesis S1** but with varying reagent feeds as noted in **TABLE 1.** The degree of cationic substitution, CS, of the QUAB^{®} 151 and the degree of hydrophobic substitution, DS, of dodecyl groups onto the dextran base polymer is reported in **TABLE 2.** The total Kjeldahl nitrogen, TKN, in the product cationic hexadecyl modified dextran polymers is also reported in **TABLE 2.**

### Synthesis S3: Synthesis of Cationic Dextran Polymer

A 500 mL, four necked, round bottom flask fitted with a rubber serum cap, a nitrogen inlet, a pressure equalizing addition funnel, a stirring paddle and motor, a subsurface thermocouple connected to a J-KEM controller and a Friedrich condenser connected to a mineral oil bubbler was charged with dextran base polymer (28.62 g; Sigma Aldrich catalog No. D4876) and deionized water (103.68 g). The contents of the flask were stirred at 70 rpm. While stirring, the head space in the flask was purged with a slow, steady flow of nitrogen (about one bubble per second) for one hour to remove any entrained oxygen in the apparatus.

The addition funnel was charged with a 70% aqueous solution of 2,3-epoxypropyltrimethylammonium chloride (27.42 g; QUAB^{®} 151 available from SKW QUAB Chemicals).

While stirring the flask contents under nitrogen, a 25% aqueous sodium hydroxide solution (5.36 g) was added to the flask contents over 1 minutes. The flask contents were then continually stirred for thirty minutes before adding the contents of the addition funnel to the flask contents dropwise over 5 minutes. The flask contents were then stirred for 5 minutes before heating the flask contents using a heating mantle with a set point temperature of 55 °C for 1.5 hours.

The flask contents were then cooled in an ice water bath while maintaining a positive nitrogen pressure in the flask. The flask contents were then neutralized by adding glacial acetic acid (3.80 g) to the flask contents. The flask contents were then stirred for 10 minutes under nitrogen. A polymer product was then recovered from the flask contents by non-solvent precipitation in methanol; roughly 700 mL of methanol was used. The methanol was then decanted off and the polymer product was placed in a dish and dried *in vacuo* at 50 °C overnight.

The polymer product recovered was sieved through a 30 mesh screen and obtained as a free-flowing white solid (25.89 g) with a volatiles content of 3.42% and an ash content (as sodium acetate) of 0.70%. The total Kjeldahl nitrogen (corrected for ash and volatiles) in the polymer product was determined to be 1.794 wt%, which corresponds to a trimethylammonium degree of substitution of 0.257.

**TABLE 1**

| **Example** | **Reagent (g)** | | | | |
|---|---|---|---|---|---|
| | **Dextran** | **NaOH** | **QUAB^{®} 151** | **Hydrophobe** | |
| | | | | **Hexadecyl Bromide** | **Dodecyl Bromide** |
| **Synthesis S1** | 20.0 | 5.0 | 17.0 | 15.3 | 0 |
| **Synthesis S2** | 20.0 | 5.0 | 17.1 | 0 | 12.5 |
| **Synthesis S3** | 28.6 | 5.4 | 27.4 | 0 | 0 |

**TABLE 2**

| **Ex.** | **hydrophobic substitution, DS** | **cationic substitution, CS** | **TKN (wt%)** |
|---|---|---|---|
| **Synthesis S1** | 0.023 | 0.217 | 1.507 |
| **Synthesis S2** | 0.007 | 0.248 | 1.729 |
| **Synthesis S3** | 0 | 0.257 | 1.794 |

### Comparative Example CF1 and Examples F1-F2: Shampoo Formulations

A shampoo formulation was prepared in each of **Comparative Example CF1** and **Examples F1-F2** having the formulation noted in **TABLE 3.** Specifically, the shampoo formulations were prepared in each of **Comparative Examples CF1** and **Examples F1-F2** using the following process: In a container, the 30 wt% aqueous solution of sodium lauryl sulfate was dissolved in 20 g of deionized water and heated to 70 °C with constant stirring. The polymer noted in **TABLE 3** was then added to the container with stirring (e.g., inventive cationic hydrophobically modified dextran prepared according to **Synthesis S1-S2,** or comparative cationic dextran prepared according to **Synthesis S3**). When the polymer was dissolved, the tetrasodium EDTA was then added to the container. Once the container contents reached 70 °C, the 45 wt% aqueous solution a portion of the PEG-150 pentaerythrityl tetrastearate and the 30 wt% aqueous solution of cocamide MEA were added to the container. Then, the 30 wt% solution of cocamidopropyl betaine was added to the container. The contents of the container were then allowed to cool. Once at room temperature, the phenoxyethanol and methylisothiazolinone preservative and the 50 wt% solids aqueous emulsion of dimethiconol and TEA-dodecylbenzenesulfonate were added to the container. The final pH of the product shampoo formulation was then adjusted to a pH of 5 using sodium hydroxide or citric acid as necessary and sufficient water was added to adjust the total formulation weight to 100 g. Additional PEG-150 pentaerythrityl tetrastearate was added to adjust the final formulation viscosity to 11,000 cP Brookfield viscosity measured using a number 6 spindle at 30 rpm under laboratory conditions.

**TABLE 3**

| **Ingredient INCI name** | **CF1** | **F1** | **F2** |
|---|---|---|---|
| | **wt% active** | | |
| Deionized water | q.s. 100 | | |
| Sodium lauryl ether sulfate¹ | 9 | 9 | 9 |
| Polymer from **Synthesis S1** | 0 | 0.3 | 0 |
| Polymer from **Synthesis S2** | 0 | 0 | 0.3 |
| Polymer from **Synthesis S3** | 0.3 | 0 | 0 |
| Tetrasodium EDTA² | 0.2 | 0.2 | 0.2 |
| Cocamide MEA³ | 1.0 | 1.0 | 1.0 |
| Cocamidopropyl Betaine⁴ | 2.1 | 2.1 | 2.1 |
| Phenoxyethanol and Methylisothiazolinone⁵ | 0.25 | 0.25 | 0.25 |
| Dimethiconol and TEA-dodecylbenzenesulfonate⁶ | 1.0 | 1.0 | 1.0 |
| PEG-150 Pentaerythrityl Tetrastearate⁷ | q.s. viscosity 11,000 cP | | |
| Sodium hydroxide or Citric acid | q.s. pH 5 | | |
| ¹ available from Stepan Company under tradename STEOL^{®} CS-130 | | | |
| ² available from The Dow Chemical Company under tradename VERSENE^{™} 220 | | | |
| ³ available from Croda Inc. under tradename INCROMIDE^{™} CMEA | | | |
| ⁴ available from Stepan Company under tradename AMPHOSOL^{®} CA | | | |
| ⁵ preservative available from The Dow Chemical Company under tradename NEOLONE^{™} PE | | | |
| ⁶ available from The Dow Chemical Company under tradename DOWSIL^{™} 1785 POE Emulsion | | | |
| ⁷ available from Croda Inc. under tradename CROTHIX^{™}-PA-(MH) | | | |

### Silicone Deposition Analysis

The silicone deposition on hair from the shampoo formulations prepared according to **Comparative Example CF1** and **Examples F1-F2** was quantified using X-ray photoelectron spectroscopy (XPS), which gives a quantitative elemental and chemical state information from the top 10 nm of the hair sample.

Hair tresses (2 g, European Virgin Brown, VB, or Bleached, B, available from International Hair Importers) were initially washed in a 9 wt% sodium lauryl sulfate solution and rinsed with water flowing at 0.4 L/min for 30 seconds. Following the initial wash step, the hair tresses were then washed with a shampoo formulation of **Comparative Example CF1** and **Examples F1-F2** by applying 0.8 g of the shampoo formulation to the hair tress and massaging in for 30 seconds on each side and then rinsing with water flowing at 0.4 L/min for 15 seconds on each side. The hair tresses were then evaluated using XPS. The XPS data were acquired from four areas per tress across a 1 cm² by 3 mm hair bundle. The instrument parameters used are provided in **TABLE 4.** The mol% of the silicon from the shampoo formulation deposited on the hair is reported in **TABLE 5.**

**TABLE 4**

| | |
|---|---|
| Instrument | K-Alpha X-ray photoelectron spectroscopy (XPS) |
| X-ray source: | Monochromatic Aluminum K-alpha |
| Analyzer Pass | 200 eV (survey spectra) |
| Energy | 20 eV (high resolution spectra) |
| Take-Off Angle | 90° |
| Auto height | on |
| Analysis Area | 400 µm oval |
| Flood Gun | on |
| Analysis Software | Casa 2.3.17 Dev. 6 3 |

**TABLE 5**

| **Shampoo Formulation** | **Hair Type** | **Si deposition (mol%)** |
|---|---|---|
| **Comparative Example CF1** | VB | 15 |
| **Comparative Example CF1** | B | 1 |
| **Example F1** | VB | 20 |
| **Example F1** | B | 14 |
| **Example F2** | VB | 17 |
| **Example F2** | B | 9 |

## Claims

1. A shampoo formulation, comprising:
a dermatologically acceptable vehicle;
a dermatologically acceptable silicone;
a dermatologically acceptable hair cleaning surfactant;
a deposition aid polymer, wherein the deposition aid polymer is a modified carbohydrate polymer, comprising a dextran base polymer functionalized with
(i) quaternary ammonium groups of formula (II) bound to a pendent oxygen on the dextran base polymer 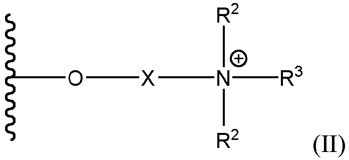 wherein 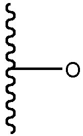 is a pendent oxygen on the dextran base polymer; wherein X is a divalent linking group; wherein each R² is independently selected from the group consisting of a C₁₋₄ alkyl group; and wherein R³ is a linear or branched C₁₋₁₆ alkyl group; and
(ii) hydrophobic substituents of formula (III) bound to a pendent oxygen on the dextran base polymer 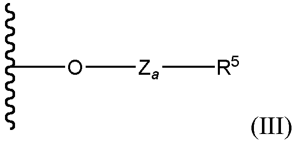 wherein 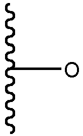 is a pendent oxygen on the dextran base polymer; wherein Z is a divalent linking group; wherein *a* is 0 or 1; and wherein each R⁵ is a linear or branched C₆₋₂₂ alkyl group.

2. The shampoo formulation of claim 1, wherein the shampoo formulation is selected from the group consisting of a shampoo and a conditioning shampoo.

3. The shampoo formulation of claim 2, wherein the deposition aid polymer has a Kjeldahl nitrogen content, TKN, of 0.5 to 4.0 wt%, wherein the Kjeldahl nitrogen content is measured using a Buchi KjelMaster K-375 automated analyzer, corrected for volatiles and ash measured as described in ASTM method D-2364.

4. The shampoo formulation of claim 3, wherein each R² is a methyl group and R³ is a methyl group.

5. The shampoo formulation of claim 3, wherein each R² is a methyl group and R³ is a linear C₈ alkyl group.

6. The shampoo formulation of claim 5, wherein the dermatologically acceptable hair cleaning surfactant includes a sodium lauryl ether surfactant selected from the group consisting of a sodium lauryl ether sulfate having an average of one -(OCH₂CH₂)- group per molecule, a sodium lauryl ether sulfate having an average of two -(OCH₂CH₂)- groups per molecule, a sodium lauryl ether sulfate having an average of three -(OCH₂CH₂)- groups per molecule and mixtures thereof.

7. The shampoo formulation of claim 3, wherein the dermatologically acceptable hair cleaning surfactant is selected from the group consisting of a sodium lauryl ether sulfate, a cocamide monoethanolamine, a cocamidopropyl betaine and mixtures thereof.

8. The shampoo formulation of claim 7, further comprising a preservative, a chelating agent and a thickener.

9. The shampoo formulation of claim 1, comprising 0.03 to 2 wt%, based on weight of the shampoo formulation, of the deposition aid polymer.

10. A method of depositing silicone on to mammalian hair, comprising:
selecting a shampoo formulation according to claim 1;
applying the shampoo formulation to mammalian hair; wherein the deposition aid polymer enhances the deposition of the dermatologically acceptable silicone from the shampoo formulation onto the mammalian hair relative to an otherwise identical formulation without the deposition aid polymer.

## Patentansprüche

1. Shampooformulierung, umfassend:
eine dermatologisch verträgliche Trägersubstanz;
ein dermatologisch verträgliches Silikon;
ein dermatologisch verträgliches Haarreinigungstensid;
ein Abscheidungshilfspolymer, wobei das Abscheidungshilfspolymer ein modifiziertes Kohlenhydratpolymer ist, umfassend ein Dextranbasispolymer, das funktionalisisert ist mit
(i) quartären Ammoniumgruppen der Formel (II), die an einen anhängenden Sauerstoff an dem Dextranbasispolymer gebunden sind 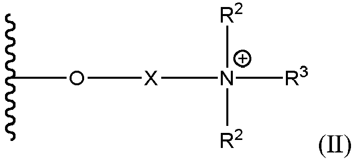 wobei 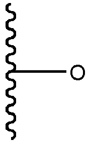 ein anhängender Sauerstoff an dem Dextranbasispolymer ist; wobei X eine zweiwertige Verknüpfungsgruppe ist; wobei jedes R² unabhängig aus der Gruppe ausgewählt ist, bestehend aus einer C₁₋₄-Alkylgruppe; und wobei R³ eine lineare oder verzweigte C₁₋₁₆-Alkylgruppe ist; und
(ii) hydrophoben Substituenten der Formel (III), die an einen anhängenden Sauerstoff an dem Dextranbasispolymer gebunden sind 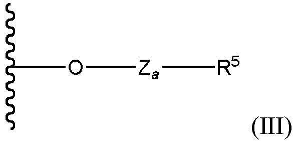 wobei 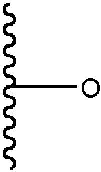 ein anhängender Sauerstoff an dem Dextranbasispolymer ist; wobei Z eine zweiwertige Verknüpfungsgruppe ist; wobei α 0 oder 1 ist; und wobei jedes R⁵ eine lineare oder verzweigte C₆₋₂₂-Alkylgruppe ist.

2. Shampooformulierung nach Anspruch 1, wobei die Shampooformulierung aus der Gruppe ausgewählt ist, bestehend aus einem Shampoo und einem Pflegeshampoo.

3. Shampooformulierung von Anspruch 2, wobei das Abscheidungshilfspolymer einen Kjeldahl-Stickstoffgehalt, TKN, von 0,5 bis 4,0 Gew.-% aufweist, wobei der Kjeldahl-Stickstoffgehalt unter Verwendung eines automatischen Buchi KjelMaster K-375-Analysators gemessen wird, korrigiert für flüchtige Substanzen und Asche, gemessen wie in ASTM-Verfahren D-2364 beschrieben.

4. Shampooformulierung nach Anspruch 3, wobei jedes R² eine Methylgruppe ist und R³ eine Methylgruppe ist.

5. Shampooformulierung nach Anspruch 3, wobei jedes R² eine Methylgruppe ist und R³ eine lineare C₈-Alkylgruppe ist.

6. Shampooformulierung nach Anspruch 5, wobei das dermatologisch verträgliche Haarreinigungstensid ein Natriumlaurylether-Tensid einschließt, ausgewählt aus der Gruppe, bestehend aus einem Natriumlaurylethersulfat, das einen Durchschnitt von einer -(OCH₂CH₂)-Gruppe pro Molekül aufweist, einem Natriumlaurylethersulfat, das einen Durchschnitt von zwei -(OCH₂CH₂)-Gruppen pro Molekül aufweist, einem Natriumlaurylethersulfat, das einen Durchschnitt von drei -(OCH₂CH₂)-Gruppen pro Molekül aufweist, und Mischungen davon.

7. Shampooformulierung nach Anspruch 3, wobei das dermatologisch verträgliche Haarreinigungstensid ausgewählt ist aus der Gruppe, bestehend aus einem Natriumlaurylethersulfat, einem Cocamid-Monoethanolamin, einem Cocamidopropylbetain und Mischungen davon.

8. Shampooformulierung nach Anspruch 7, ferner umfassend ein Konservierungsmittel, einen Chelatbildner und ein Verdickungsmittel.

9. Shampooformulierung nach Anspruch 1, umfassend zu 0,03 bis 2 Gew.-%, bezogen auf das Gewicht der Shampooformulierung, das Abscheidungshilfspolymer.

10. Verfahren zum Abscheiden von Silikon auf Säugetierhaar, umfassend:
Auswählen einer Shampooformulierung nach Anspruch 1;
Auftragen der Shampooformulierung auf Säugetierhaar; wobei das Abscheidungshilfspolymer die Abscheidung des dermatologisch verträglichen Silikons aus der Shampooformulierung auf das Säugetierhaar im Vergleich zu einer ansonsten identischen Formulierung ohne das Abscheidungshilfspolymer verbessert.

## Revendications

1. Formulation de shampoing, comprenant :
un véhicule dermatologiquement acceptable ;
un silicone dermatologiquement acceptable ;
un agent tensioactif de nettoyage pour cheveux dermatologiquement acceptable ;
un polymère d'aide au dépôt, dans laquelle le polymère d'aide au dépôt est un polymère glucidique modifié, comprenant un polymère à base de dextrane fonctionnalisé avec
(i) des groupes ammonium quaternaires de formule (II) liés à un oxygène greffé sur le polymère à base de dextrane 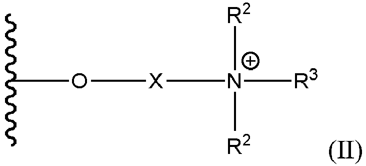 dans laquelle 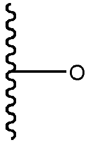 est un oxygène greffé sur le polymère de base de dextrane ; dans laquelle X est un groupe pontant divalent ; dans laquelle chaque R² est choisi indépendamment dans le groupe constitué d'un groupe alkyle en C₁₋₄ ; et dans laquelle R³ est un groupe alkyle en C₁₋₁₆ linéaire ou ramifié ; et
(ii) des substituants hydrophobes de formule (III) liés à un oxygène pendant sur le polymère à base de dextrane 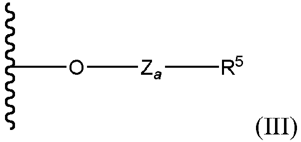 dans laquelle 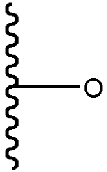 est un oxygène greffé sur le polymère de base de dextrane ; dans laquelle Z est un groupe pontant divalent ; dans laquelle α vaut 0 ou 1 ; et dans laquelle chaque R⁵ est un groupe alkyle en C₆₋₂₂ linéaire ou ramifié.

2. Formulation de shampoing selon la revendication 1, dans laquelle la formulation de shampoing est choisie dans le groupe constitué d'un shampoing et d'un shampoing revitalisant.

3. Formulation de shampoing selon la revendication 2, dans laquelle le polymère d'aide à dépôt a une teneur en azote Kjeldahl, TKN, de 0,5 à 4,0 % en poids, dans laquelle la teneur en azote Kjeldahl est mesurée à l'aide d'un analyseur automatisé Buchi KjelMaster K-375, corrigée des matières volatiles et des cendres mesurées comme décrit dans le procédé ASTM D-2364.

4. Formulation de shampoing selon la revendication 3, dans laquelle chaque R² est un groupe méthyle et R³ est un groupe méthyle.

5. Formule de shampoing selon la revendication 3, dans laquelle chaque R² est un groupe méthyle et R³ est un groupe alkyle linéaire en C₈.

6. Formulation de shampoing selon la revendication 5, dans laquelle l'agent tensioactif de nettoyage de cheveux dermatologiquement acceptable comporte un agent tensioactif lauryl éther de sodium choisi dans le groupe constitué d'un lauryl éther de sodium sulfate ayant une moyenne d'un groupe -(OCH₂CH₂)- par molécule, un lauryl éther de sodium sulfate ayant une moyenne de deux groupes - (OCH₂CH₂)- par molécule, un lauryléther de sodium sulfate ayant une moyenne de trois groupes -(OCH₂, CH₂)- par molécule, et des mélanges de ceux-ci.

7. Formulation de shampoing selon la revendication 3, dans laquelle l'agent tensioactif de nettoyage de cheveux dermatologiquement acceptable est choisi dans le groupe constitué d'un sulfate lauryl éther de sodium, d'un monoéthanolamine de cocamide, d'une cocamidopropylbétaïne et de mélanges de ceux-ci.

8. Formulation de shampoing selon la revendication 7, comprenant en outre un conservateur, un agent chélatant et un épaississant.

9. Formulation de shampoing selon la revendication 1, comprenant 0,03 à 2 % en poids, sur la base du poids de la formulation de shampoing, du polymère d'aide au dépôt.

10. Procédé de dépôt de silicone sur des cheveux de mammifère, comprenant :
la sélection d'une formulation de shampoing selon la revendication 1 ;
l'application de la formulation de shampoing sur des cheveux de mammifère ; dans lequel le polymère d'aide au dépôt améliore le dépôt du silicone dermatologiquement acceptable de la formulation de shampooing sur les poils de mammifères par rapport à une formulation par ailleurs identique sans le polymère d'aide au dépôt.
